Europäisches Patentamt

(19) European Patent Office

Office européen des brevets

(11) Publication number : **0 300 899 B1**

(12) **EUROPEAN PATENT SPECIFICATION**

(45) Date of publication of patent specification :
**18.09.91 Bulletin 91/38**

(21) Application number : **88401876.3**

(22) Date of filing : **20.07.88**

(51) Int. Cl.⁵ : **A61M 19/00, A61M 5/165**

(54) **Filter device for peridural anaesthesia.**

(30) Priority : **23.07.87 FR 8710479**

(43) Date of publication of application :
**25.01.89 Bulletin 89/04**

(45) Publication of the grant of the patent :
**18.09.91 Bulletin 91/38**

(84) Designated Contracting States :
**BE DE FR GB IT SE**

(56) References cited :
**DE-A- 1 816 972**
**GB-A- 2 043 478**
**US-A- 4 326 957**
**US-A- 4 336 036**

(73) Proprietor : **MILLIPORE S.A.**
**Zone Industrielle**
**F-67120 Molsheim (FR)**

(72) Inventor : **Lemonnier, Jean**
**8 rue de la Fontaine**
**F-78110 Le Vesinet (FR)**

(74) Representative : **Rinuy, Santarelli**
**14, avenue de la Grande Armée**
**F-75017 Paris (FR)**

EP 0 300 899 B1

Jouve, 18, rue Saint-Denis, 75001 PARIS

# Description

The present invention concerns a filter device for peridural or epidural anaesthesia.

This type of local/regional anaesthesia is increasingly used nowadays to treat pain, especially for chronic illnesses, surgical operations and childbirth.

The practioner has first to find the peridural space containing the rachidian nerve to be anaesthetised by inserting the tip of a bevelled peridural needle upwardly between the second and third lumbar vertebrae. The tip enters the peridural space delimited by the dura mater and the walls of the rachidian canal into which a flexible catheter will be introduced through the needle to enable the anaesthetic to be injected.

To prevent transfer of the anaesthetic causing bacterial or microparticle contamination (glass, rubber, etc), it is essential to use a filter device between the syringe and the catheter.

Two different types of filter device have been used hitherto for peridural anaesthesia.

A first type consists of a circular filter membrane the edge of which is welded to a plastics material casing in two parts, placed on either side of the membrane ; each of these two parts comprises an enclosure with an inlet or outlet orifice the axis of which is perpendicular to the plane of the filter membrane.

Although this first type of filter was entirely satisfactory from the technical point of view, it did not suit in practice because of its pear-shaped structure, which prevented it being attached conveniently and comfortably to the body of the patient under treatment.

A second type of filter has been launched onto the market which differs from the previous type in that the axes of the inlet and outlet orifices leading to and from the spaces situated on either side of the filter membrane are not perpendicular but rather parallel to the plane of the membrane which is itself parallel to the filter casing.

However, in this second type of filter the inlet and outlet orifices project from the same side of the membrane, which has a two-fold disadvantage.

On the one hand, this kind of arrangement means that only one side of the filter, that opposite the side from which the membrane access orifices project, can be attached to the body of the patient.

On the other hand as both orifices are situated on the same side of the membrane there has to be provided for one of them a U-shaped passage providing access to the opposite side of the membrane, which requires the presence of a third plastics material member, of circular ring shape, to delimit this access channel. A structure of this kind with three plastics material components increases the unit cost of manufacturing this type of filter and also, because of the U-shaped channel providing access to one side of the membrane, produces a head loss which reduces the sensitivity of the equipment when used by the anaesthetist.

At last, GB-A-2,043,478 discloses a filter device wherein a membrane is sealed along a groove formed in the annular shoulder of a shallow disc-shaped plastic base having an inlet and an outlet provided on opposite sides of the shoulder so that fluid passing through the filter device must pass through the membrane and a substantially flat cap is applied to the plastic base for completing the filter device. In this prior device, wherein one support surface for the filter membrane is free of any relief member projecting beyond the support surface, the membrane, which is not in contact with the substantially flat cap, is supported only on one side and the filter device is therefore not reversible.

An object of the present invention is a filter device whose structure makes it possible to overcome these disadvantages.

The flat, reversible and disposable filter device in accordance with the invention for peridural anaesthesia comprises a rigid casing consisting of only two plastics material parts which are welded to each other and disposed on either side of a filter membrane, each plastics material part comprising an inlet or outlet bore communicating with a space comprising membrane support members and one of the two support surfaces for the surface membrane is free of any relief members projecting beyond said support surface.

This filter device is characterised by the fact that the membrane support members of each of the two plastics material parts have a circular support surface defining a plane at an acute angle to the axes of the inlet and outlet bores.

By virtue of the inclination of the filter membrane, the access orifices situated on either side of the membrane can be disposed on the filter device without them projecting beyond the edges of the membrane.

A structure of this kind makes the filter device entirely reversible so that it can be placed on the body of the patient either way up, which eliminates the possibility of positioning errors.

Also the fact that one of the membrane support surfaces provided in one of the two plastics material parts does not comprise any relief members projecting beyond this surface makes it possible to weld the membrane to this surface using an automatic device working from a continuous strip of filter material from which the membrane is cut out.

Finally the circular shape of the filter membrane support surfaces and the inclination of the membrane relative to the direction of movement of the filtered liquid make it possible to avoid the formation of any air pocket a source of bubbles which can cause unwanted disturbance to the flow of the fluid after it passes through the filter device in accordance with the invention.

The present invention will now be described in one specific embodiment shown in the appended drawings in which :

— figure 1 is a view of the filter device in accordance with the invention in cross-section on the line 1-1 in figure 2 ;

— figure 2 is a plan view of the device from figure 1 ;

— figures 3 and 4 are respectively a view in cross-section on the line 3-3 in figure 4 and a plan view of the lower part of the plastics material casing ; and

— figures 5 and 6 are respectively a view in cross-section on the line 5-5 in figure 6 including the filter membrane and a bottom view without the filter membrane of the upper part of the plastics material casing of the filter device in accordance with the invention.

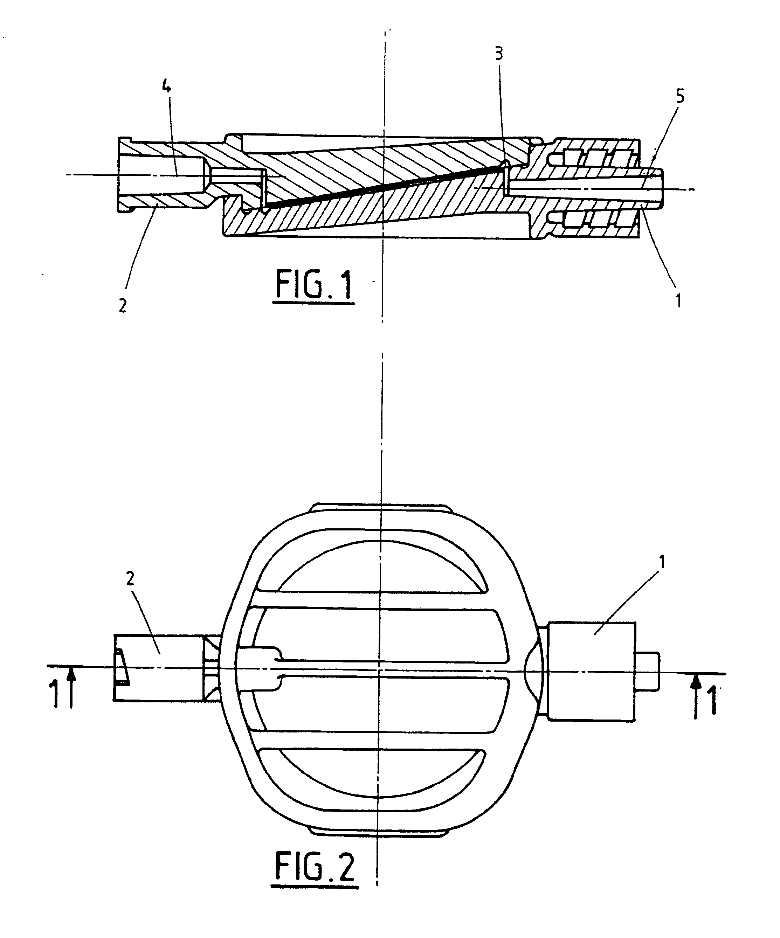

The filter device for peridural anaesthesia shown in figures 1 and 2 comprises a casing consisting of two plastics material parts 1 and 2 between which is disposed a filter membrane 3.

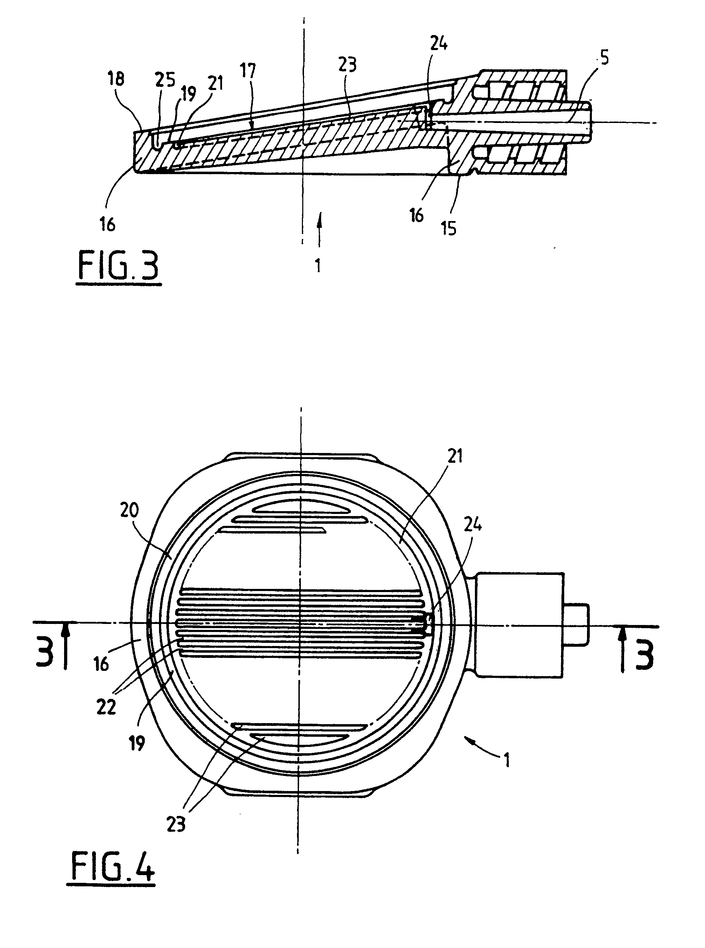

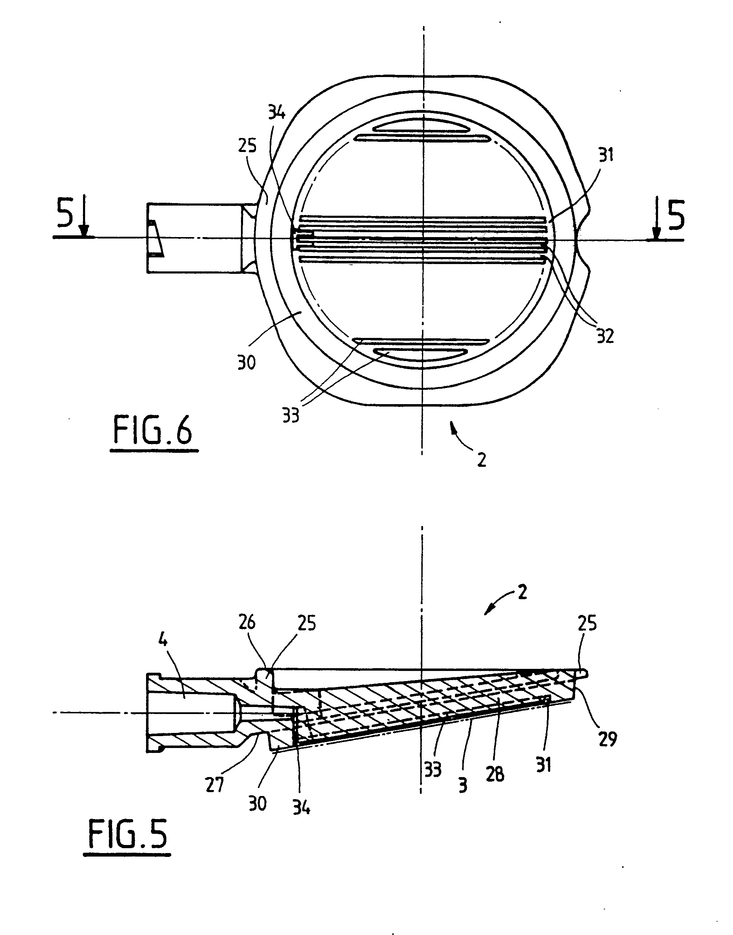

The lower part or base 1 is shown in figures 3 and 4 and the upper part or lid 2 is shown in figures 5 and 6.

As shown in figure 3 in particular the base 1 comprises a substantially cylindrical vertical wall 16 having a lower and 15 situated in a horizontal plane and an upper end 18 situated in a plane at an acute angle alpha to the horizontal.

This angle alpha is preferably between 5 and 15° and in the example shown in figures 1 through 6 is equal to 10°.

The base 1 also comprises a bottom 17 the plane of which is at the same acute angle as the upper end 18 of the wall 16 so as to form a female member.

The upper surface of the bottom 17, which forms an inclined plane, comprises a plane peripheral ring 19 delimited by an external circular groove 20 and an internal circular groove 21.

All of the surface located inside the plane ring 19 comprises parallel longitudinal grooves 22 which open into the internal groove 21 and are delimited by parallel longitudinal ribs 23.

The tops of the ribs 23 are in substantially the same plane as the plane peripheral ring 19 so as to form a lower inclined support surface for the filter membrane 3.

The space defined by the internal circular groove 21 and the longitudinal grooves 22 constitutes the sole means giving access to the lower surface of the filter membrane 3.

Such a structure allows the air to escape through the filter membrane, when the liquid is fed, before the membrane, wet by the liquid, becomes gas-tight.

This disposition allows, due to the absence of any chamber in permanent contact with the surface of the filter membrane, to avoid the formation of an air bubble, known under the name of air lock, which reduces the filtering surface available and consequently the efficiency of the filter device.

The base 1 also comprises an outlet bore 5 the axis of which, as shown in figure 3, is parallel to the plane of the lower end 15 and which is conical in shape with a double external screwthread forming a conventional "LUER" type connector as used for catheters.

The bore 5 passes through the wall 16 and communicates at right angles with a feed channel 24 which opens directly into the internal circular groove 21.

The feed channel 24 preferably has a U-shaped tranverse cross-section (see figure 4) in which the base of the U opens into the groove 21 and the two branches of the U, the length of which can increase as said section moves closer to the groove 21, open into two parallel longitudinal grooves 22.

The upper part or lid 2 of the filter device in accordance with the invention comprises, as shown in figure 5, a vertical wall 25 having a plane upper end 26 situated in a horizontal plane and a lower end 27 situated in a plane at the same acute angle alpha to the horizontal as the base 1.

The lid 2 also comprises a bottom 28 the plane of which is at the same acute angle as the lower end 27 of the wall 25 and which is joined to the latter by a substantially cylindral wall 29 the diameter of which is slightly less than that of the wall 25 so as to form a male member the dimensions of which correspond approximately to the female member of the base 1.

The length of this vertical cylindrical wall 29 is such that the lower surface of the bottom 28 does not comprise any relief members projecting beyond this surface.

The lower surface of the bottom 28, which forms an inclined plane (see figure 5), comprises a plane peripheral ring 30 delimited by the vertical wall 29 and by an internal circular groove 31. All of the surface situated inside the plane ring 30 comprises parallel longitudinal grooves 32 which open into the internal grooves 31 and are delimited by parallel longitudinal ribs 33.

The tops of the ribs 33 are in substantially the same plane as the plane peripheral rings 30 so as to form an upper inclined support surface for the filter membrane 3.

In the embodiment shown in figures 1 through 6 the axial rib 23 of the base 1 and the axial rib 33 of the lid 2 are situated in the same plane and extend in the same direction as the axes of the inlet bore 4 and the outlet bore 5, so as to facilitate the flow of the liquid to be filtered.

The space defined by the internal circular groove 31 and the longitudinal grooves 32 constitutes the sole means giving access to the upper surface of the filter membrane 3.

This structure of the upper inclined support surface for the filter membrane in the lid 2 is quite similar to the one of lower inclined support surface for the membrane in the base 1.

The lid 2 also comprises an inlet bore 4 the axis of which, as shown in figure 5, is parallel to the plane of the upper end 26 and which is conical in shape to serve as a connector for the end of a syringe.

The bore 4 passes through the wall 25 and communicates at right angles with a feed channel 34 which opens directly into the internal circular groove 31.

Like the channel 24, this feed channel 34 preferably has a U-shaped transverse cross-section (see figure 6) in which the base of the U opens into the groove 31 and the two branches of the U, the length of which can increase towards the groove 31, open into two parallel longitudinal grooves 32.

This particular shape of the transverse cross-section of the channel 24 of the base 1 and the channel 34 of the lid 2 makes it possible to mould these two parts 1 and 2 from plastics material without producing any burrs between the corresponding bore and the channel, which is extremely important for safe peridural anaesthesia, especially on the downstream side of the flow of liquid filtered in accordance with the invention.

As shown in figure 5, the circular filter membrane 3 is welded to the plane peripheral ring 30 of the lid 2 and this welding may easily be performed by automatic means working from a continuous strip of filter material, given the absence of any relief members projecting beyond the lower surface of the bottom 28.

After welding the filter membrane 3 to the rings 30, the base 1 is placed and aligned on the lid 2 so that these two plastics members, which constitute the casing of the filter device in accordance with the invention, can be ultrasonically welded to each other in their final position along the cylindrical wall 29.

After this welding, the filter membrane 3 is sandwiched between the ribs 23 and 33, the tops of which are supporting it on each side. Due to this welding and these support members, located as well upstream as downstream, the filter membrane is maintained flat and cannot loose its shape, rendering the filter device perfectly reversible.

The materials of the filter membrane 3 and the plastics material parts 1 and 2 are conventional and do not form any part of the present invention.

However, the filter membrane, which generally has a pore diameter between 0.1 and 0.5 microns, may be made from a cellulose ester, a polyolefin or polytetrafluorethylene and the plastics material of the envelope may be polyvinylchloride, a polyolefin or styrene-acrylonitrile resin.

The filter device according to the invention is particularly suitable for hydrophilic filter membranes and allows notably the use of such membranes which are very brittle, such as those made of cellulosic esters.

It should be noted that all the welds necessary for assembling the claimed filter device are performed externally of the filter surface, which makes it possible to avoid burrs or molten material debris interfering with the filtration process.

Also, the path of the filtered liquid is designed to minimise head losses and to obtain an average flowrate by gravity which is higher than that obtained with filter devices previously available through commercial channels for peridural anaesthesia with the same filter membrane surface area and characteristics.

These enhanced properties are obtained by virtue of a structure that is simple to manufacture as there are fewer component parts to be welded together, safer in use as the sensitivity that the anaesthetist requires is retained and more practical as it is more compact and entirely reversible.

The filter unit, due to its structure, may operate, whatever be its position, whether horizontal, vertical or other and it can be used therefore directly in contact with a patient, without to be maintained compulsorily in a vertical position.

## Claims

1. Flat, reversible, disposable filter device for peridural anaesthesia comprising a rigid casing consisting of only two plastics material parts (1 and 2) which are welded together, disposed on either side of a filter membrane (3) and each comprising an inlet bore (4) or outlet bore (5) communicating with a space comprising support members for the membrane (3) and wherein one of the two support surfaces for the filter membrane is free of any relief members projecting beyond said support surface, characterized in that the membrane support members of each of the two plastics material parts (1 and 2) have a circular support surface defining a plane at an acute angle to the axes of the inlet bore (4) and the outlet bore (5).

2. Filter device according to claim 1, characterised in that the filter membrane (3) is welded at its edge to a plane peripheral ring of the support surface on which there are no relief members.

3. Filter device according to claim 1 or claim 2, characterised in that the acute angle between the membrane support surface and the axis of the inlet and outlet orifices (4 and 5) is between 5 and 15° and is preferably 10°.

4. Filter device according to any of claims 1 through 3, characterised in that the membrane support surface of at least one plastics material part (1 and 2), and preferably of both, comprises parallel longitudinal ribs (23, 33) in the direction of the axes of the inlet bore (4) and the outlet bore (5).

5. Filter device according to claim 4, characterised in that at least one of the support surfaces for the membrane (3) comprises longitudinal grooves (22, 32) and a peripheral circular groove (21, 31) for flow of the liquid to be filtered through the filter membrane.

6. Filter device according to claim 5, characterised in that the inlet bore (4) or outlet bore (5), and preferably both, communicates with the corresponding peripheral circular groove (21, 31) through a feed channel (24, 34) opening directly into said groove (21, 31).

7. Filter device according to claim 6, characterised in that the feed channel (24, 34) is perpendicular to the axis of the inlet bore (4) or outlet bore (5).

8. Filter device according to claim 6 or claim 7, characterised in that the feed channel (24, 34) opens directly into one of the peripheral circular grooves (21, 31), and preferably into both, and has a U-shaped transverse cross-section.

9. Filter device according to claim 8, characterised in that the feed channel (24, 34) has a U-shaped transverse cross-section in which the base of the U opens into the peripheral circular groove (21, 31) and the two branches of the U preferably open into two longitudinal grooves (22, 32).

## Patentansprüche

1. Flacher, umkehrbar einsetzbarer Einmalfilter für die Periduralanästhesie mit einem starren Gehäuse, das nur aus zwei zusammengeschweißten Kunststoffteilen (1 und 2) besteht, die beidseits einer Filtermembrane (3) angeordnet sind und deren jedes eine Einlaß- (4) oder eine Auslaßbohrung (5) aufweist, die mit einem Raum mit Stützgliedern für die Membrane (3) in Verbindung steht, wobei eine der zwei Stützflächen für die Filtermembrane frei von erhabenen, über diese Stützfläche vorstehenden Teilen ist, **dadurch gekennzeichnet,** daß die Stützglieder für die Membrane jedes der beiden Kunststoffteile (1 und 2) eine kreisförmige Stützfläche aufweisen, die eine in einem spitzen Winkel zu den Achsen der Einlaßbohrung (4) und der Auslaßbohrung (5) liegende Ebene festlegt.

2. Filter nach Anspruch 1, dadurch gekennzeichnet, daß die Filtermembrane (3) an ihrem Rand mit einem ebenen Umfangsring der Stützfläche, die keine erhabenen Teile aufweist, verschweißt ist.

3. Filter nach Anspruch 1 oder 2, dadurch gekennzeichnet, daß der spitze Winkel zwischen der Stützfläche für die Membrane und der Achse der Einlaß- und Auslaßöffnungen (4 und 5) zwischen 5° und 15°, vorzugsweise 10°, beträgt.

4. Filter nach einem der Ansprüche 1 bis 3, dadurch gekennzeichnet, daß die Stützfläche für die Membrane an wenigstens einem Kunststoffteil (1 und 2), vorzugsweise an beiden Kunststoffteilen, parallele Längsrippen (23, 33) in Richtung der Achsen der Einlaßbohrung (4) und der Auslaßbohrung (5) aufweist.

5. Filter nach Anspruch 4, dadurch gekennzeichnet, daß wenigstens eine der Stützflächen für die Membrane (3) Längsnuten (22, 32) und eine kreisförmige Umfangsnut (21, 31) zum Durchfluß der durch die Filtermembrane zu filternden Flüssigkeit aufweist.

6. Filter nach Anspruch 5, dadurch gekennzeichnet, daß die Einlaßbohrung (4) oder die Auslaßbohrung (5), vorzugsweise beide, mit der entsprechenden kreisförmigen Umfangsnut (21, 31) über einen Zuführkanal (24, 34) in Verbindung stehen, der direkt in diese Nut (21, 31) mündet.

7. Filter nach Anspruch 6, dadurch gekennzeichnet, daß der Zuführkanal (24, 34) senkrecht zur Achse der Einlaßbohrung (4) oder Auslaßbohrung (5) verläuft.

8. Filter nach Anspruch 6 oder 7, dadurch gekennzeichnet, daß der Zuführkanal (24, 34) direkt in eine der kreisförmigen Umfangsnuten (21, 31), vorzugsweise aber in beide, mündet und einen U-förmigen Querschnitt aufweist.

9. Filter nach Anspruch 8, dadurch gekennzeichnet, daß der Zuführkanal (24, 34) einen U-förmigen Querschnitt aufweist, bei dem die Basis des U in die kreisförmige Umfangsnut (21, 31) und die zwei Schenkel des U vorzugsweise in zwei Längsnuten (22, 32) münden.

## Revendications

1. Dispositif de filtration plat, réversible, à jeter après usage, pour anesthésie péridurale, comportant un boîtier rigide constitué de seulement deux pièces (1 et 2) en matière plastique qui sont soudées entre elles, disposées de chaque côté d'une membrane filtrante (3) et comportant chacune une lumière d'entrée (4) ou une lumière de sortie (5) communiquant avec un espace comprenant des éléments de support pour la membrane (3), et dans lequel l'une des deux surfaces de support pour la membrane filtrante est libre de tous éléments en relief faisant saillie au-delà de ladite surface de support, caractérisé en ce que les éléments de support de la membrane de chacune des deux pièces (1 et 2) en matière plastique ont une surface de support circulaire définissant un plan formant un angle aigu avec les axes de la lumière d'entrée (4) et de la lumière de sortie (5).

2. Dispositif de filtration selon la revendication 1, caractérisé en ce que la membrane filtrante (3) est soudée par son bord à un anneau périphérique plan de la surface de support sur laquelle il n'y a aucun élément en relief.

3. Dispositif de filtration selon la revendication 1 ou la revendication 2, caractérisé en ce que l'angle aigu entre la surface de support de la membrane et l'axe des orifices d'entrée et de sortie (4 et 5) est compris entre 5 et 15° et est de préférence de 10°.

4. Dispositif de filtration selon l'une quelconque des revendications 1 à 3, caractérisé en ce que la surface de support de la membrane d'au moins une pièce (1 et 2) en matière plastique, et de préférence des deux, comporte des nervures longitudinales parallèles (23, 33) dans la direction des axes de la lumière d'entrée (4) et de la lumière de sortie (5).

5. Dispositif de filtration selon la revendication 4, caractérisé en ce qu'au moins l'une des surfaces de support pour la membrane (3) comporte des gorges longitudinales (22, 32) et une gorge périphérique circulaire (21, 31) pour l'écoulement du liquide à filtrer à travers la membrane filtrante.

6. Dispositif de filtration selon la revendication 5, caractérisé en ce que la lumière d'entrée (4) ou la lumière de sortie (5), et de préférence les deux, communiquent avec la gorge circulaire périphérique correspondante (21, 31) par un canal d'alimentation (24, 34) débouchant directement dans ladite gorge (21, 31).

7. Dispositif de filtration selon la revendication 6, caractérisé en ce que le canal d'alimentation (24, 34) est perpendiculaire à l'axe de la lumière d'entrée (4) ou de la lumière de sortie (5).

8. Dispositif de filtration selon la revendication 6 ou la revendication 7, caractérisé en ce que le canal d'alimentation (24, 34) débouche directement dans l'une des gorges circulaires périphériques (21, 31), et de préférence dans les deux, et présente une section transversale de forme en U.

9. Dispositif de filtration selon la revendication 8, caractérisé en ce que le canal d'alimentation (24, 34) présente une section transversale de forme en U dans laquelle la base du U débouche dans la gorge circulaire périphérique (21, 31) et les deux branches du U débouchent de préférence dans deux gorges longitudinales (22, 32).

FIG.1

FIG.2

FIG.3

FIG.4

FIG.6

FIG.5